# EUROPEAN PATENT APPLICATION

(11) **EP 4 545 556 A1**
(43) Date of publication of application: **30.04.2025**
(21) Application number: 23383102.3
(22) Date of filing: 27.10.2023
(51) Int. Cl.: C07K 14/47, A23J 1/20, A23L 33/10, A23J 3/08

(54) **PRODUCTION OF RECOMBINANT PROTEINS**

(71) Applicant: Real Deal Milk S.L., 08010 Barcelona (ES)
(72) Inventor: Oberlaender, Gabriel Mora, 08010 Barcelona (ES); Mirasol, Esther Paulo, 08010 Barcelona (ES); Ramos, Cristina Corral, 08010 Barcelona (ES); Mercado, Sergi Monforte, 08010 Barcelona (ES); Gilabert, Omar Tomás, 08010 Barcelona (ES)
(74) Representative: HGF

(57) **Abstract**

A recombinant fusion protein is provided comprising a first protein and a caseinomacropeptide (CMP), or a fragment thereof. The recombinant fusion protein may be produced by culturing a host cell comprising a nucleic acid encoding the protein. The recombinant fusion protein finds use in the production of recombinant proteins.

## Description

### Field of the invention

The present invention relates to a recombinant fusion protein, and methods for preparing proteins which utilise a recombinant fusion protein. More particularly, the invention relates to a recombinant fusion protein comprising a caseinomacropeptide.

### Background

Proteins are greatly complex molecules that can be suitable for many different applications. Recombinant proteins enable the production of a huge range of useful proteins without the need for animal agriculture. Current uses of recombinant proteins include food production, consumer goods, the agricultural industry (animal feeds to support nutrition and gut health), and the medical industry (enzymes, hormones, antibodies, and therapeutic molecules). However, due to pooryields recombinant proteins have struggled commercially. Most approaches taken to increase protein yields aim to increase the cell-specific productivity by genetic engineering or optimizing the culture conditions. Examples of current methods to increase yields include the engineering of production lines and expression vectors, increasing the copy number of the expression cassette, optimization of the cell culture medium and optimisation of fermentation strategies.

Milk (e.g. cows' milk) is an oil-in-water emulsion which comprises dissolved carbohydrates, minerals, lipids and proteins. The proteins present in milk include caseins (α-S1, α-S2, β and κ-caseins) and whey proteins (including β-lactoglobulin (BLG), α-lactalbumin, serum albumin and immunoglobulins).

Demand for cow's milk and dairy products is expected to keep increasing due to increased reliance on these products in developing countries as well as growth in human population. Relying on animal agriculture to meet growing demand for food is not a sustainable solution.

β-Lactoglobulin (BLG) is the main protein (ca. 50%) in the whey fraction of milk from cows and other ruminants (e.g. buffalo, sheep, goat). Due to their excellent nutritional quality and functional properties, whey proteins are highly valued ingredients. They find applications as a principal component of nutritional products (e.g. in protein supplements for sports nutrition or clinical nutrition) or as a secondary ingredient in diverse food preparations (e.g. dairy products, smoothies, beverages, sauces, dips, soups, bakery goods, protein bars, desserts, confectionery, processed meats) modifying either their nutritional profile or physical properties (e.g. acting as a stabilizer, an emulsifier or altering viscosity) (Goulding, D.A. et al. Chapter 2 - Milk proteins: An overview, Eds.: Boland, M. and Singh, H. Milk Proteins (Third Edition), Academic Press, 2020, Pages 21-98).

Current methods for production of BLG through animal agriculture are associated with a very high environmental impact, as well as with health dangers inherent to industrialized animal agriculture. However, despite the elevated ecological cost, these methods have low economic cost, resulting in relatively cheap production of the protein.

BLG is amenable to production through precision fermentation. In contrast to animal agriculture, production through fermentation has the potential to enormously reduce ecological impact as well to eliminate health dangers (no antibiotics, no zoonotic diseases), but, with current technology, the economic cost of production is higher than that of animal agriculture and the product is, therefore, not competitive.

The production of proteins, including milk proteins such as BLG, through the expression of recombinant DNA in a heterologous host system is thus desirable for multiple ecological, health and animal welfare reasons. For it to be economically viable, the process must occur with high titre (g protein / L culture) and/or high productivity (g protein / number of viable cells in culture).

Well established methods to achieve high level titre and productivity of recombinant proteins in heterologous expression systems, such as expression under a strong promoter or insertion of multiple copies of the recombinant DNA, are not equally effective for all proteins.

Accordingly, there is a need to provide proteins in a more sustainable and/or economically viable manner.

### Summary of the invention

According to a first aspect of the present invention there is provided a recombinant fusion protein comprising:
(a) a first protein; and
(b) a caseinomacropeptide (CMP), or a fragment thereof,
wherein the first protein is not a casein.

The invention thus provides a recombinant fusion protein comprising a first protein fused to a caseinomacropeptide (CMP), or a fragment thereof, wherein the first protein is not a casein.

According to a second aspect of the invention there is provided a nucleic acid molecule comprising a sequence encoding the recombinant fusion protein according to the first aspect of the invention.

According to a third aspect of the invention, there is provided a vector comprising the nucleic acid molecule of the second aspect of the invention.

According to a fourth aspect of the invention, there is provided a host cell comprising the nucleic acid molecule of the second aspect of the invention, or the vector of the third aspect of the invention.

In a fifth aspect, the invention provides a method of producing a recombinant protein, the method comprising culturing the host cell of the fourth aspect of the invention under conditions suitable for expression of the recombinant fusion protein of the first aspect.

In a sixth aspect, the invention provides a method of making a food composition, the method comprising:
(i) providing a recombinant fusion protein according to the first aspect of the invention;
(ii) separating the first protein from the CMP or fragment thereof; and
(iii) using the separated first protein to make a food composition.

In a further aspect, the invention provides a recombinant protein obtained by the method of the fifth aspect.

In yet a further aspect, the invention provides a food composition obtained by the method of the sixth aspect.

In another aspect, the invention provides the use of the recombinant fusion protein of the first aspect in the production of a food composition.

Embodiments of the invention will now be described with reference to the accompanying figures in which:
Figure 1 is a schematic drawing of a recombinant fusion protein in accordance with embodiments of the invention;
Figure 2 is a schematic drawing of a recombinant protein, e.g. β-lactoglobulin (BLG), obtained from cleavage of the recombinant fusion protein of Figure 1;
Figure 3 is a comparative example showing the quantity of recombinant α-S1 casein, α-S2 casein, β-casein and κ-casein expressed under a strong promoter in *Pichia pastoris*; and
Figure 4 is a comparative example showing the quantity of casein (CSN) - caseinomacropeptide (CMP) recombinant fusion proteins expressed using the same promoter, host cell and conditions as in the example shown in Figure 3;
Figure 5 is an image of a Western blot showing 6xHIS tagged BLG or BLG-linker-CMP, wherein the linker comprises either a chymosin or an enteropeptidase recognition and cleavage sequence; and
Figure 6 is a graph showing the titre for 6xHIS tagged BLG or BLG-linker-CMP, wherein the linker comprises either a chymosin or an enteropeptidase recognition and cleavage sequence.

In this specification, amino acids are designated by the one-letter code, for example, G stands for glycine, P stands for proline, R stands for arginine, etc.

The present invention provides a recombinant fusion protein comprising:
(a) a first protein; and
(b) a caseinomacropeptide (CMP), or a fragment thereof,
wherein the first protein is not a casein.

It will be understood that neither the first protein, nor the recombinant fusion protein as a whole, comprises a full-length casein, e.g. α-S1 casein, α-S2 casein or β-casein or κ-casein.

While some proteins can be expressed recombinantly in good yield, it is known that other proteins are not expressed in high yield in a heterologous host. Well established methods to achieve high level titre and productivity of recombinant proteins in heterologous expression systems, such as expression under a strong promoter or insertion of multiple copies of the recombinant DNA, are not equally effective for all proteins. For example, α-S1 casein, α-S2 casein and β-casein do not respond well to these strategies. Unexpectedly, the inventors have found that by fusing CMP to proteins, even those (e.g. β-lactoglobulin) which are already expressed in heterologous hosts in good yield, the yield of those proteins can be increased.

It is known in the art to classify proteins into the following categories: contractile, structural, storage, antibodies, enzymes, hormonal and transport.

In some embodiments, the recombinant protein is an antibody, an enzyme or a hormonal protein. In some embodiments, the recombinant protein is not an antibody, an enzyme or a hormonal protein.

In some embodiments, the protein is a contractile, structural, storage or transport protein.

Contractile proteins include actin, myosin and tropomyosin.

Structural proteins include actin, actinin, biglycan, collagen, decorin, fibrinogen, fibrin, elastin keratin, mucin, myelin associated glycoprotein, myosin, spectrin, tropomyosin, troponin, tubulin, vimentin and vitronectin.

Transport proteins include haemoglobin, cytochromes, myoglobin.

In some embodiments the recombinant protein is an animal protein (e.g. a milk protein or a meat protein) or a plant protein.

In some embodiments the animal protein is a meat protein. The meat protein may be selected from myosin, actin, myoglobin, collagen and elastin.

In some embodiments, the protein is a storage protein. These are proteins evolved to fulfil the nutritional requirements of organisms through different developmental stages and are the protein sources animals have adapted to and incorporated into their diets. The use of storage proteins as basis for a therapeutic recombinant protein is therefore expected to reduce the disruptive effect of diet replacement. Table 1 presents a non-exhaustive list of examples of storage proteins, together with representative organisms and accession numbers.

The storage protein may be an animal storage protein, or it may be a plant storage protein. It will be appreciated that the protein may be derived from any suitable species. Table 1 presents a non-exhaustive list of examples of storage proteins.

In some embodiments the animal protein is a milk protein. The milk protein may be selected from whey protein, lactalbumin (e.g. α-lactalbumin), lactoglobulin (e.g. β-lactoglobulin).

In some embodiments the first protein is glycodelin.

In some embodiments the animal protein is an egg protein. The egg protein may be selected from ovalbumin, ovaltransferrin, ovamucoid, flavoprotein, avidin, biotin, high-density lipoprotein (HDL), low-density lipoprotein (LDL), phosvitin, livetin, globulin and ovomucin.

In some embodiments, the plant storage protein is a protein derived from soy, wheat, maize, barley, oat, sorghum, pea, rice or cacao.

In some embodiments, the plant storage protein is selected from glycinin (e.g. glycinin G1, G2, G3, G4 or G5), beta-conglycinin (e.g. beta-conglycinin alpha 1, beta-conglycinin beta 1, beta-conglycinin alpha 2, or beta-conglycinin beta 2), alpha-gliadin, beta-gliadin, gamma-gliadin, avenin-like a1, avenin-like b1, B-1 hordein, B-3 hordein, C hordein, gamma hordein 1, Glutelin-2, 22 kDa alpha-zein 16, 22 kDa alpha-zein 14, alpha kafirin, avenin (e.g. avenin-3, avenin-E), vicilin, albumin (e.g. albumin-1a, albumin-2), or glutelin (e.g. glutelin type I or type-B 2).

**Table 1**

| **Protein** | **Type** | **Representative organism** | **Representative UniProt Accession #** |
|---|---|---|---|
| Beta Lactoglobulin (BLG) | Whey (milk) | e.g. Cow (Bos taurus) | P02754 |
| Alpha Lactalbumin (LALBA) | Whey (milk) | e.g. Cow (Bos taurus) | P00711 |
| Ovalbumin | Albumin | e.g. Chicken (Gallus gallus) | P01012 |
| Glycinin G1 | Globulin (11s) | Soy (Glycine max) | P04776 |
| Glycinin G2 | Globulin (11s) | Soy (Glycine max) | P04405 |
| Glycinin G3 | Globulin (11s) | Soy (Glycine max) | P11828 |
| Glycinin G4 | Globulin (11s) | Soy (Glycine max) | P02858 |
| Glycinin G5 | Globulin (11s) | Soy (Glycine max) | P04347 |
| Beta-conglycinin alpha 1 | 7S seed storage family | Soy (Glycine max) | P11827 |
| Beta-conglycinin beta 1 | 7S seed storage family | Soy (Glycine max) | P25974 |
| Beta-conglycinin alpha 2 | 7S seed storage family | Soy (Glycine max) | P0DO15 |
| Beta-conglycinin beta 2 | 7S seed storage family | Soy (Glycine max) | F7J077 |
| Alpha/beta-gliadin | Prolamin | Wheat (Triticum aestivum) | P18573 |
| Gamma-gliadin | Prolamin | Wheat (Triticum aestivum) | P21292 |
| Avenin-like a1 | Prolamin | Wheat (Triticum aestivum) | Q2A784 |
| Avenin-like b1 | Prolamin | Wheat (Triticum aestivum) | Q2A783 |
| B-1 hordein | Prolamin | Barley (Hordeum vulgare) | P06470 |
| B-3 hordein | Prolamin | Barley (Hordeum vulgare) | P06471 |
| C hordein | Prolamin | Barley (Hordeum vulgare) | P06472 |
| Gamma hordein 1 | Prolamin | Barley (Hordeum vulgare) | P17990 |
| Glutelin-2 | Zein (Prolamin) | Maize (Zea mays) | P04706 |
| 22 kDa alpha-zein 16 | Zein (Prolamin) | Maize (Zea mays) | P04700 |
| 22 kDa alpha-zein 14 | Zein (Prolamin) | Maize (Zea mays) | P04698 |
| Alpha kafirin | Prolamin | Sorghum (Sorghum bicolor) | B1PHV3 |
| Avenin | Prolamin | Oat (Avena sativa) | P27919 |
| Avenin - 3 | Prolamin | Oat (Avena sativa) | P80356 |
| Avenin - E | Prolamin | Oat (Avena sativa) | Q09114 |
| Vicilin | 7S seed storage family | Pea (Pisum sativum) | P13918 |
| Albumin - 1a | Albumin | Pea (Pisum sativum) | P62926 |
| Albumin - 2 | Albumin | Pea (Pisum sativum) | P08688 |
| Vicilin | 7S seed storage family | Cacao (Theobroma cacao) | Q43358 |
| Glutelin type I | Globulin (11s) | Rice (Oryza sativa) | P07728 |
| Glutelin type-B 2 | Globulin (11s) | Rice (Oryza sativa) | Q02897 |

Preferably, the first protein is selected from: beta-lactoglobulin, ovalbumin, hordein, conglycinin and alpha-lactalbumin. More preferably, the recombinant protein is beta-lactoglobulin (BLG).

Among the factors determining cost of production through precision fermentation, titre (g protein / L culture) and productivity (g protein / number of viable cells in culture) are high impact ones. Surprisingly, it has been found that expression of recombinant BLG as a fusion protein with the N-terminal portion of kappa casein (the caseinomacropeptide - CMP) can increase yield more than 10 fold over expression of recombinant BLG alone and can therefore have a strong impact on production cost. Like the protein BLG, the peptide CMP is a component of whey. It is therefore a safe, digestible and highly nutritious molecule. In consequence, a BLG-CMP fusion protein can in many applications be used in place of a BLG protein. Nevertheless, for applications where BLG alone is required, the components of the fusion protein can be linked by a cleavable linker allowing removal of the peptide and regeneration of BLG from the fusion protein.

The first protein, e.g. BLG, may comprise or be constituted by an amino acid sequence which is at least 70%, at least 75%, at least 80%, at least 85%, at least 90%, at least 95%, at least 96%, at least 97%, at least 98%, or at least 99% identical to that of the equivalent wild-type protein, e.g. BLG.

The first protein may be derived from any suitable species. For example, the first protein, e.g. BLG, may be that of a ruminant, such as cow and sheep, or non-ruminants, such as pig or horse. In some embodiments, the BLG is bovine BLG.

In some embodiments, the BLG comprises or is constituted by an amino acid sequence which is at least 70%, at least 75%, at least 80%, at least 85%, at least 90%, at least 95%, at least 96%, at least 97%, at least 98% or at least 99% identical to the sequence of bovine (*Bos taurus*) BLG (Uniprot accession no. P02754).

A representative sequence for bovine BLG, excluding the native secretion signal, is shown below and is referred to herein as SEQ ID NO. 1. Thus, in some embodiments, the first protein comprises or is constituted by an amino acid sequence which is at least 70%, at least 75%, at least 80%, at least 85%, at least 90%, at least 95%, at least 96%, at least 97%, at least 98% or at least 99% identical to SEQ ID NO. 1.

LIVTQTMKGLDIQKVAGTWYSLAMAASDISLLDAQSAPLRVYVEELKPTPEGDLEILLQKWENGE
CAQKKIIAEKTKIPAVFKIDALNENKVLVLDTDYKKYLLFCMENSAEPEQSLACQCLVRTPEVDDE
ALEKFDKALKALPMHIRLSFNPTQLEEQCHI (SEQ ID NO. 1)

The term "identity", with reference to amino acid and nucleic acid sequences, is used herein to describe the level of similarity between two sequences. Sequence similarity or identity may be determined using standard techniques known in the art, including, but not limited to, the local sequence identity algorithm of Smith & Waterman, Adv. Appl. Math. 2, 482 (1981), by the sequence identity alignment algorithm of Needleman & Wunsch, J Mol. Biol. 48,443 (1970), by the search for similarity method of Pearson & Lipman, Proc. Natl. Acad. Sci. USA 85, 2444 (1988), by computerized implementations of these algorithms (GAP, BESTFIT, FASTA, and TFASTA in the Wisconsin Genetics Software Package, Genetics Computer Group, 575 Science Drive, Madison, Wl), the Best Fit sequence program described by Devereux et al., Nucl. Acid Res. 12, 387-395 (1984), or by inspection. Another suitable algorithm is the BLAST algorithm, described in Altschul et al., J Mol. Biol. 215, 403-410, (1990) and Karlin et al., Proc. Natl. Acad. Sci. USA 90, 5873-5787 (1993).

The recombinant fusion protein of the invention comprises a first protein which is fused, directly or indirectly, to a caseinomacropeptide (CMP), or a fragment thereof.

As used herein, the term "caseinomacropeptide" or "CMP" refers to the C-terminal portion of κ-casein (i.e. the portion which is released upon cleavage of κ-casein by chymosin, which may also be referred to as the "CMP domain") but excludes the chymosin recognition sequence. As is known in the art, bovine chymosin cuts κ-casein between an F residue and an M residue (residues 105 and 106 in bovine κ-casein) to release an N-terminal portion (para-kappa casein) and a C-terminal portion (comprising the caseinomacropeptide). The FM motif is contained within a recognition sequence, which in bovine κ-casein is HPHPHLSFMAIPPK (SEQ ID NO:2). Although, post-cleavage, a part of this recognition sequence resides in the C-terminal portion, this part of the recognition sequence is not considered to form a part of the CMP as defined in the context of this specification. However, in some embodiments the recombinant fusion protein comprises a linker which may include some or all of the recognition sequence.

In some embodiments, the recombinant fusion protein, or the CMP or fragment thereof, comprises a sequence of no more than 150, no more than 120, no more than 100, no more than 90, no more than 80, no more than 75, no more than 72, no more than 70, no more than 65, no more than 64, no more than 60, or no more than 58 consecutive amino acids derived from the C-terminus of κ-casein. In other words, the recombinant fusion protein, or the CMP or fragment thereof, does not comprise the full length κ-casein protein.

In some embodiments, the recombinant fusion protein, or the CMP or fragment thereof, comprises a sequence of 72 consecutive amino acids derived from the C-terminus of κ-casein.

In some embodiments, the recombinant fusion protein, or the CMP or fragment thereof, comprises a sequence of at least 20, at least 25, at least 30, at least 35, at least 40, at least 45 or at least 50 consecutive amino acids derived from the C-terminus of κ-casein.

In some embodiments, the recombinant fusion protein, or the CMP or fragment thereof, comprises a sequence of from 20 to 120, from 25 to 110, from 30 to 100, from 35 to 90, from 40 to 85, from 50 to 80, from 55 to 75, from 60 to 70 consecutive amino acids derived from the C-terminus of κ-casein.

The terms "cleavage site" and "recognition sequence" may be used interchangeably herein to refer to a sequence or chemical motif which is recognised by an enzyme or a chemical capable of cleaving the recombinant fusion protein so as to separate the first protein (e.g. BLG) from the CMP or fragment thereof.

The glycosylated form of CMP may also be referred to in the art as "GMP" or "gCMP" (glyco-caseino-macropeptide), while the non-glycosylated form is sometimes referred to as "aCMP" (aglyco-caseino-mactopeptide). It is also known in the art to use the term "CMP" for a heterogeneous mixture of non-glycosylated peptides and peptides with different degrees of glycosylation. In the context of the present specification, the term "CMP" is used to refer to both the non-glycosylated peptide and to glycosylated forms, unless otherwise stated.

In some embodiments, the CMP or fragment thereof increases the hydrophilicity of the recombinant fusion protein relative to the hydrophilicity of the first protein alone. The CMP is a highly hydrophilic sequence, particularly in its glycosylated states. This is evident from the CMP sequence itself. Thus, in some embodiments the CMP or fragment thereof is capable of conferring on the recombinant fusion protein an increased hydrophilicity, relative to the hydrophilicity of the (equivalent) first protein alone. In other words, the hydrophilicity of the recombinant fusion protein may be higher than the hydrophilicity of the (equivalent) first protein alone. In some embodiments, increasing hydrophilicity can result in more efficient biosynthesis, less intracellular degradation, better host-cell fitness and/or more efficient secretion.

It will be understood that increasing the hydrophilicity of the recombinant fusion protein is equivalent to decreasing its hydrophobicity. In some embodiments the CMP or fragment thereof decreases the hydrophobicity of the recombinant fusion protein relative to the hydrophobicity of the first protein alone. Thus, the CMP or fragment thereof may be capable of conferring on the recombinant fusion protein a reduced hydrophobicity, relative to the hydrophobicity of the (equivalent) first protein alone. In other words, the hydrophobicity of the recombinant fusion protein may be lower than the hydrophobicity of the (equivalent) first protein alone.

The hydrophobicity of the recombinant fusion protein and/or the first protein may be determined by Hydrophobic Partition, as described in Nakai S. 2003 Measurement of Protein Hydrophobicity; Current Protocols in Food Analytical Chemistry 9(1), Basic Protocol 5.

In some embodiments, the CMP or fragment thereof is capable of increasing the solubility of the recombinant fusion protein in a medium, relative to the solubility of the (equivalent) first protein alone in the same medium. In other words, the solubility of the recombinant fusion protein in a medium may be greater than the solubility of the (equivalent) first protein alone in the medium.

The effect of fusion to the CMP or fragment thereof on the solubility of a first protein may be determined according to the following protocol:
1. Prepare multiple separate aliquots of recombinant fusion protein and first protein alone at the same concentration (e.g. 2.7% w/w) in demineralised water adjusted to high pH with NaOH (e.g. pH 11);
2. Adjust the pH of each aliquot to a different (lower) value (e.g. decrease in 1 pH unit increments down to pH 1) with HCl, and incubate (e.g. 30 min with stirring) for equilibration;
3. Determine the resulting concentration in each condition by centrifugation of the equilibrated sample (e.g. 4000 g, 15 min) and of the original solution (pH 11), then remove the supernatant and add 1 volume of 2x SDS PAGE buffer to the supernatant. Run the samples on an SDS-PAGE gel and divide the intensity of the band obtained for each condition by the intensity of the band obtained at pH 11.

In some embodiments, the CMP or fragment thereof is capable of increasing the yield of the recombinant fusion protein relative to the yield of the (equivalent) first protein alone when expressed under identical conditions (e.g. from the same expression cassette, in the same host, under the same environmental conditions). In other words, the yield of the recombinant fusion protein may be greater than the yield of the (equivalent) first protein alone when expressed under identical conditions.

In some embodiments, the yield of a recombinant fusion protein comprising a first protein is at least 1.5, at least 2, at least 2.5, at least 3, at least 3.5, at least 4, at least 4.5, at least 5, at least 6, at least 7, at least 8, at least 9 or at least 10 times greater than the yield of the first protein alone when expressed under identical conditions. In some embodiments, the yield of a recombinant fusion protein comprising a first protein is at least 150%, at least 200%, at least 300%, at least 400%, at least 500%, at least 600%, at least 800% or at least 1000% of the yield of the first protein alone when expressed under identical conditions.

In some embodiments, the CMP or fragment thereof comprises a sequence of at least 20, at least 25, at least 30, at least 35, at least 40, at least 45 or at least 50 consecutive amino acids.

In some embodiments, the CMP or fragment thereof comprises at least 3, at least 4, at least 5 glycosylation sites. In some embodiments, the CMP or fragment thereof comprises 6 glycosylation sites.

In some embodiments, the CMP comprises an N-terminal or a C-terminal truncation, relative to the full length CMP (as defined herein). In some embodiments, the CMP comprises both an N-terminal and a C-terminal truncation, relative to the full length CMP (as defined herein).

In some embodiments the CMP or fragment thereof comprises:
(i) the amino acid sequence:
   X₃SGX₂PX₁ X₁X₁PTX₃X₂X₃X₃X₂S X₁X₃X₃X₁X₃X₂X₃X₁PX₂ X₃ (SEQ ID NO:3)
   wherein
   each X₁ is independently selected from S and T,
   each X₂ is independently selected from D and E, and
   each X₃ is independently selected from A, I, L and V; or
(ii) an amino acid sequence which is at least 80%, at least 85%, at least 90% or at least 95% identical to SEQ ID NO: 3.

In some embodiments, the CMP or fragment thereof comprises an amino acid sequence which is at least 80%, at least 85%, at least 90% or at least 95% identical to SEQ ID NO: 3, with the proviso that at least 3, at least 4 or at least 5 of the X₁ residues are retained. In some embodiments, all of the X₁ residues are retained.

In some embodiments the CMP or fragment thereof comprises:
(i) an amino acid sequence selected from:
   X₃SGX₂PX₁ X₁X₁PTX₃X₂X₃X₃X₂S X₁X₃X₃X₁X₃X₂X₃X₁PX₂ X₃X₃X₂SPPX₂X₃NT X₃QX₃X₁STX₃X₃ (SEQ ID NO:4); and
   KNQX₂KTX₂X₃PX₁ X₃NTX₃X₃SGX₂PX₁ X₁X₁PTX₃X₂X₃X₃X₂S X₁X₃X₃X₁X₃X₂X₃X₁PX₂ X₃ (SEQ ID NO:5), or
(ii) an amino acid sequence which is at least 70%, at least 75%, at least 80%, at least 85%, at least 90% or at least 95% identical to SEQ ID NO: 4 or SEQ ID NO:5.

In some embodiments, the CMP or fragment thereof comprises an amino acid sequence which is at least 70%, at least 75%, at least 80%, at least 85%, at least 90% or at least 95% identical to SEQ ID NO: 4, with the proviso that at least 3, at least 4, at least 5 or at least 6 of the X₁ residues are retained. In some embodiments, all of the X₁ residues are retained.

In some embodiments, the CMP or fragment thereof comprises an amino acid sequence which is at least 70%, at least 75%, at least 80%, at least 85%, at least 90% or at least 95% identical to SEQ ID NO: 5, with the proviso that at least 3, at least 4, at least 5 or at least 6 of the X₁ residues are retained. In some embodiments, all of the X₁ residues are retained.

In some embodiments the CMP or fragment thereof comprises:
(i) the amino acid sequence 
KNQX2KTX2X3PX1 X3NTX3X3SGX2PX1 X1X1PTX3X2X3X3X2S X1X3X3X1X3X2X3X1PX2
X3X3X2SPPX2X3NT X3QX3X1STX3X3 (SEQ ID NO:6);
 or
(ii) an amino acid sequence which is at least 70%, at least 75%, at least 80%, at least 85%, at least 90% or at least 95% identical to SEQ ID NO: 6.

In some embodiments, the CMP or fragment thereof comprises an amino acid sequence which is at least 70%, at least 75%, at least 80%, at least 85%, at least 90% or at least 95% identical to SEQ ID NO: 6, with the proviso that at least 3, at least 4, at least 5, at least 6 or at least 7 of the X₁ residues are retained. In some embodiments, all of the X₁ residues are retained.

In some embodiments the CMP or fragment thereof comprises:
(i) the amino acid sequence ASGEPT STPTIEAVES TVATLEASPE V (SEQ ID NO:7); or
(ii) an amino acid sequence which is at least 80%, at least 85%, at least 90% or at least 95% identical to SEQ ID NO: 7.

In some embodiments, the CMP or fragment thereof comprises an amino acid sequence which is at least 80%, at least 85%, at least 90% or at least 95% identical to SEQ ID NO: 7 with the proviso that at least 3, at least 4 or at least 5 glycosylation sites are retained (i.e. present). In some embodiments, 6 glycosylation sites are retained.

In some embodiments the CMP or fragment thereof comprises:
(i) an amino acid sequence selected from:
   ASGEPT STPTIEAVES TVATLEASPE VIESPPEINT VQVTSTAV (SEQ ID NO:8); and
   KNQDKTEIPT INTIASGEPT STPTIEAVES TVATLEASPE V (SEQ ID NO:9), or
(ii) an amino acid sequence which is at least 70%, at least 75%, at least 80%, at least 85%, at least 90% or at least 95% identical to SEQ ID NO: 8 or SEQ ID NO:9.

In some embodiments, the CMP or fragment thereof comprises an amino acid sequence which is at least 70%, at least 75%, at least 80%, at least 85%, at least 90% or at least 95% identical to SEQ ID NO: 8, with the proviso that at least 3, at least 4, at least 5 or at least 6 glycosylation sites are retained (i.e. present). In some embodiments, 7 glycosylation sites are retained.

In some embodiments, the CMP or fragment thereof comprises an amino acid sequence which is at least 70%, at least 75%, at least 80%, at least 85%, at least 90% or at least 95% identical to SEQ ID NO:9, with the proviso that at least 3, at least 4, at least 5 or at least 6 glycosylation sites are retained (i.e. present). In some embodiments, 7 glycosylation sites are retained.

In some embodiments the CMP or fragment thereof comprises or consists of:
(i) the amino acid sequence
   KNQDKTEIPT INTIASGEPT STPTIEAVES TVATLEASPE VIESPPEINT VQVTSTAV (SEQ ID NO:10); or
(ii) an amino acid sequence which is at least 70%, at least 75%, at least 80%, at least 85%, at least 90%, at least 92%, at least 95%, at least 96% or at least 98% identical to SEQ ID NO: 10.

In some embodiments the CMP or fragment thereof comprises (an amino acid sequence which is at least 70%, at least 75%, at least 80%, at least 85%, at least 90%, at least 92%, at least 95%, at least 96% or at least 98% identical to SEQ ID NO: 10, with the proviso that at least 3, at least 4, at least 5, at least 6 or at least 7 glycosylation sites are retained (i.e. present). In some embodiments, 8 glycosylation sites are retained.

In some embodiments, the CMP comprises or is constituted by an amino acid sequence which is at least 70%, at least 75%, at least 80%, at least 85%, at least 90%, at least 95%, at least 96%, at least 97%, at least 98%, or at least 99% identical to the CMP of cow (*Bos taurus*) κ-casein (Uniprot accession no. P02668). The cow κ-casein may be variant B, or it may be the reference variant A or any one of the other variants (B2, E, G, or F).

The amino acid sequence of the CMP from cow κ-casein protein (variant B) is designated herein as SEQ ID NO:10.

The CMP, or fragment thereof, may be derived from any suitable mammal. In some embodiments, the CMP comprises or is constituted by an amino acid sequence which is at least 70%, at least 75%, at least 80%, at least 85%, at least 90%, at least 95%, at least 96%, at least 97%, at least 98%, or at least 99% identical to the wild-type CMP of κ-casein from goat, sheep, camel, donkey, horse, human, rat, mouse, guinea pig, pig, water buffalo, rabbit, whale (e.g. fin whale, beluga whale), giraffe or elephant. The accession numbers of κ-casein for exemplary species are shown in Table 2.

**Table 2**

| **Species** | **K casein accession no. (uniprot)** |
|---|---|
| Cow (*Bos taurus*) | P02668 |
| Goat | P02670 |
| Sheep | P02669 |
| Camel | P79139 |
| Donkey | B1PLB8 |
| Human | P07498 |
| Horse | P82187 |
| Rat | P04468 |
| Mouse | P06796 |
| Guinea pig | P19442 |
| Pig | P11841 |
| Domestic water buffalo | P11840 |
| Rabbit | P33618 |
| Fin whale | Q27952 |
| Beluga whale | A0A2Y9M6S7 |
| Giraffe | Q28417 |
| African elephant | G3SNT0 |

As used herein, the term "fusion" will be understood to mean that the first protein and the caseinomacropeptide (CMP) or fragment thereof are joined directly or indirectly via peptide bonds such that they form two portions of the same recombinant protein.

In some embodiments, the recombinant fusion protein comprises a linker between the first protein (e.g. BLG) and the CMP or fragment thereof.

The linker may comprise an amino acid sequence of from 3 to 50, from 5 to 40, from 8 to 30, from 10 to 20 or from 12 to 15 residues in length.

In some embodiments, the linker comprises a cleavage site. Advantageously, the cleavage site can be used to cleave the recombinant fusion protein so as to separate the first protein from the CMP or fragment thereof. The cleavage site may be a chemical cleavage site or an enzyme cleavage site.

In some embodiments, the cleavage site is an enzyme cleavage site. The enzyme cleavage site may be a hydrolase cleavage site (such as a protease cleavage site) or a peptide lyase cleavage site (such as an amidine lyase cleavage site).

In some embodiments the cleavage site is a protease cleavage site. The cleavage site may be a chymosin cleavage site, or an enteropeptidase cleavage site.

A suitable enteropeptidase cleavage site may be that of bovine enteropeptidase (Uniprot accession number P98072).

In some embodiments, the cleavage site is a chymosin cleavage site. The chymosin cleavage site may be that of any mammal. For example, the chymosin cleavage site may be bovine, goat, sheep, or camel. In some embodiments, the cleavage site is derived from the same species as the CMP.

In some embodiments the chymosin cleavage site comprises the amino acid sequence HLSFXAIP (SEQ ID NO.11), wherein X is L, M or F. Cleavage occurs between the F and X residues.

Some additional residues are believed to make the interaction with the enzyme more stable. Therefore, in some embodiments the chymosin cleavage site comprises the sequence HPHPHLSFMAIPPK (SEQ ID NO.2).

In some embodiments, the chymosin cleavage site comprises the sequence FLQKQQYGISSKFX wherein X is selected from: W, Y, F, C, R, I, M, H, L, V, K, N, T or A (SEQ ID NO.12). Preferably, X is W, Y or F.

In some embodiments, the chymosin cleavage site comprises the sequence RPRPRPSFIAIPPK (SEQ ID NO.13).

Other suitable cleavage sites which may be incorporated into the recombinant fusion protein will be known to those skilled in the art, such as those described in WO2022/072718.

The first protein (e.g. BLG) may be located at or towards the N-terminal end of the recombinant fusion protein, and the CMP or fragment thereof may be located at or towards the C-terminal end of the recombinant fusion protein. Alternatively, the first protein may be located at or towards the C-terminal end of the recombinant fusion protein, and the CMP or fragment thereof may be located at or towards the N-terminal end of the recombinant fusion protein.

In some embodiments, the first protein is located at or towards the N-terminal end, and the CMP or fragment thereof at or towards the C-terminal end of the recombinant fusion protein.

The recombinant fusion protein may further comprise additional features, such as one or more tags (e.g. a HIS tag or a FLAG tag), and/or a signal sequence.

It will be appreciated that the components of the recombinant fusion protein (i.e. the first protein, the CMP or fragment thereof, and any tag(s) and/or signal sequences, if present) may be directly joined to each other via peptide bonds, or they may be joined via one or more linkers.

In some embodiments, the recombinant fusion protein comprises a signal sequence or a fragment thereof. As is known in the art, a signal sequence (also referred to as a "signal peptide", "leader sequence", "signal leader", "targeting signal", "localization signal" or "localization sequence") is a short amino acid sequence that is normally present at the N-terminus of a protein, and which prompts the producing cell to translocate the protein through a membrane. A secretory signal sequence prompts translocation through the membrane of the endoplasmic reticulum (in eukaryotic organisms) and therefore into the secretory pathway. Signal sequences may be cleaved during or after translocation of the protein through the target membrane to generate a free signal peptide and a mature protein. Complex leader sequences can comprise multiple processing (i.e. cleavage) positions. A first (N-terminal) portion, which strictly speaking is the localization sequence, is referred to as pre-sequence (or "pre-signal", "pre-region", "pre-peptide") and is cleaved during translocation or immediately after translocation. A further portion, referred to as pro-sequence (or "pro-signal", "pro-peptide", "pro-region"), is believed to modulate progression through the pathway and is cleaved at later stages of transit through the secretory pathway. Incomplete processing of a complex leader sequence may result in the secreted protein retaining some amino acids from the pro-sequence at its N terminus. Thus, in some embodiments the recombinant fusion protein comprises at its N terminus at least some amino acids from the pro-region of the signal sequence.

The signal sequence may be any suitable signal that is known in the art. The skilled person is capable of selecting a signal sequence which is appropriate for the host cell in which the recombinant fusion protein is intended to be expressed.

In some embodiments, the signal sequence is selected from: S. *cerevisiae* α-mating factor (MF-a) signal sequence or a chimera, fragment or variant (e.g. comprising point mutations) thereof.

In some embodiments, the signal sequence is the *S*. *cerevisiae* α-mating factor (MF-a) signal sequence.

In some embodiments, the signal sequence is a truncated version of MF-a signal sequence, e.g. MF-α(57-70).

In some embodiments, the signal sequence is a chimeric signal sequence comprising the pre-region of the *S*. *cerevisiae* Ost1 signal sequence and the pro-region of the *S*. *cerevisiae* MF-a signal sequence.

In some embodiments, the signal sequence comprises a sequence which is at least 90%, at least 95%, at least 96%, at least 97%, at least 98% or at least 99% identical to SEQ ID NO. 14.

MRFPSIFTAVLFAASSALAAPVNTTTEDETAQIPAEAVIGYSDLEGDFDVAVLPFSNSTNNGLLFIN
TTIASIAAKEEGVSLEKREAEA (SEQ ID NO:14).

In some embodiments, the signal sequence comprises a sequence which is at least 90%, at least 95%, at least 96%, at least 97%, at least 98% or at least 99% identical to SEQ ID NO. 22.

MRFPSIFTAVLFAASSALAAPVNTTTEDETAQIPAEAVIGYSDLEGDFDVAVLPFSASIAAKEEGV
SLEKREAEA (SEQ ID NO. 22).

In some embodiments, the recombinant fusion protein comprises or has the structure, from N-terminus to C-terminus:
Signal sequence (or fragment thereof) - optional tag - first protein - linker - CMP (or fragment thereof) - optional tag, wherein the linker comprises a cleavage site (e.g. a chymosin cleavage site).

In some embodiments, the recombinant fusion protein comprises or has the structure, from N-terminus to C-terminus:
Signal sequence (or fragment thereof) - first protein - linker - CMP (or fragment thereof), wherein the linker comprises a cleavage site (e.g. a chymosin cleavage site).

In some embodiments, the recombinant fusion protein comprises or has the structure, from N-terminus to C-terminus:
First protein - linker - CMP (or fragment thereof), wherein the linker comprises a cleavage site (e.g. a chymosin cleavage site).

In an embodiment, the recombinant fusion protein comprises or consists of an amino acid sequence which is at least 70%, at least 75%, at least 80%, at least 85%, at least 90%, at least 95%, at least 96%, at least 97%, at least 98% or at least 99% identical to SEQ ID NO: 15, SEQ ID NO:16 or SEQ ID NO:17. In an embodiment, the recombinant fusion protein comprises or consists of an amino acid sequence according to SEQ ID NO:15, SEQ ID NO:16 or SEQ ID NO:17. In these embodiments, the recombinant fusion protein comprises BLG fused via a linker to the CMP peptide of bovine k-casein, wherein a HIS tag is provided at the C-terminus of the fusion protein (underlined). However, it will be appreciated that other suitable tags could be used.

In the sequence of SEQ ID NO:15 and SEQ ID NO:16 the linker comprises the bovine chymosin cleavage site (underlined). In the sequence of SEQ ID NO:17, the linker comprises the enteropeptidase cleavage sequence (underlined).

The sequence of SEQ ID NO: 15 comprises the *S*. *cerevisiae* α-mating factor Δ57-70 (MF-α57-70) signal sequence at its N-terminus (underlined). During secretion of the recombinant fusion protein by the host cell, this signal sequence is cleaved, leaving behind a small fragment thereof on the secreted protein (underlined). The secreted protein is represented by SEQ ID NO:16.

MRFPSIFTAVLFAASSALAAPVNTTTEDETAQIPAEAVIGYSDLEGDFDVAVLPFSASIAAKEEGV
SLEKREAEALIVTQTMKGLDIQKVAGTWYSLAMAASDISLLDAQSAPLRVYVEELKPTPEGDLEIL
LQKWENGECAQKKIIAEKTKIPAVFKIDALNENKVLVLDTDYKKYLLFCMENSAEPEQSLACQCLV

RTPEVDDEALEKFDKALKALPMHIRLSFNPTQLEEQCHIHPHPHLSFMAIPPKKNQDKTEIPTINTI
ASGEPTSTPTIEAVESTVATLEASPEVIESPPEINTVQVTSTAVGSGSHHHHHH (SEQ ID NO: 15).

EAEALIVTQTMKGLDIQKVAGTWYSLAMAASDISLLDAQSAPLRVYVEELKPTPEGDLEILLQKW
ENGECAQKKIIAEKTKIPAVFKIDALNENKVLVLDTDYKKYLLFCMENSAEPEQSLACQCLVRTPE
VDDEALEKFDKALKALPMHIRLSFNPTQLEEQCHIHPHPHLSFMAIPPKKNQDKTEIPTINTIASGE
PTSTPTIEAVESTVATLEASPEVIESPPEINTVQVTSTAVGSGSHHHHHH (SEQ ID NO:16).

EAEALIVTQTMKGLDIQKVAGTWYSLAMAASDISLLDAQSAPLRVYVEELKPTPEGDLEILLQKW
ENGECAQKKIIAEKTKIPAVFKIDALNENKVLVLDTDYKKYLLFCMENSAEPEQSLACQCLVRTPE
VDDEALEKFDKALKALPMHIRLSFNPTQLEEQCHIDDDDKKNQDKTEIPTINTIASGEPTSTPTIEA
VESTVATLEASPEVIESPPEINTVQVTSTAVGSGSHHHHHH (SEQ ID NO:17).

The invention further provides a nucleic acid molecule comprising a sequence encoding a recombinant fusion protein as described herein.

The nucleic acid molecule may be DNA or RNA. In some embodiments the nucleic acid molecule is DNA.

In some embodiments the nucleic acid molecule comprises a sequence according to SEQ ID NO:18. This sequence encodes an amino acid sequence according to SEQ ID NO: 16.

In some embodiments the nucleic acid molecule comprises a sequence according to SEQ ID NO:19. This sequence encodes an amino acid sequence according to SEQ ID NO: 17.

The portions of the sequences encoding the secretion signal sequence, the linker and the tag are underlined in the sequences shown. The sequences have been codon optimized for expression in *Pichia pastoris.*

ATGAGATTTCCATCTATTTTCACTGCTGTTTTGTTTGCTGCTTCTTCTGCTTTGGCTGCTCCA
GTTAACACTACTACTGAAGATGAGACTGCTCAAATTCCTGCTGAAGCTGTTATTGGTTACTCT
GATTTGGAGGGAGATTTTGATGTTGCTGTTTTGCCTTTCTCTGCTTCTATCGCTGCTAAGGA
AGAGGGTGTTTCTTTGGAAAAGAGAGAAGCTGAGGCTTTGATTGTTACTCAAACTATGAAGG
GTTTGGATATCCAAAAGGTTGCTGGTACTTGGTACTCTTTGGCTATGGCTGCTTCTGATATTT
CTTTGTTGGATGCTCAATCTGCTCCATTGAGAGTTTATGTTGAAGAGTTGAAACCAACTCCT
GAAGGAGATTTGGAAATTTTGTTGCAAAAGTGGGAAAACGGAGAGTGTGCTCAAAAGAAAAT
TATCGCTGAAAAGACTAAGATCCCTGCTGTTTTTAAAATCGATGCTTTGAACGAGAATAAGGT
TTTGGTTTTGGATACTGATTACAAGAAATATTTGTTGTTCTGTATGGAAAACTCTGCTGAACC

AGAGCAATCTTTGGCTTGTCAATGTTTGGTTAGAACTCCTGAAGTTGATGATGAAGCTTTGG
AGAAGTTTGATAAAGCTTTGAAGGCTTTGCCAATGCATATCAGATTGTCTTTCAATCCTACTC
AATTGGAAGAGCAATGTCACATTCATCCACACCCTCATTTGTCTTTCATGGCTATTCCACCTA
AGAAAAACCAAGATAAGACTGAAATCCCAACTATTAATACTATTGCTTCTGGAGAGCCAACTT
CTACTCCTACTATTGAAGCTGTTGAGTCTACTGTTGCTACTTTGGAAGCTTCTCCTGAAGTTA
TTGAGTCTCCACCTGAGATTAATACTGTTCAAGTTACTTCTACTGCTGTTGGTTCTGGATCCC
ATCACCATCACCATCACTAA (SEQ ID NO. 18).

ATGAGATTTCCATCTATTTTCACTGCTGTTTTGTTTGCTGCTTCTTCTGCTTTGGCTGCTCCA
GTTAACACTACTACTGAAGATGAGACTGCTCAAATTCCTGCTGAAGCTGTTATTGGTTACTCT
GATTTGGAGGGAGATTTTGATGTTGCTGTTTTGCCTTTCTCTGCTTCTATCGCTGCTAAGGA
AGAGGGTGTTTCTTTGGAAAAGAGAGAAGCTGAGGCTTTGATTGTTACTCAAACTATGAAGG
GTTTGGATATCCAAAAGGTTGCTGGTACTTGGTACTCTTTGGCTATGGCTGCTTCTGATATTT
CTTTGTTGGATGCTCAATCTGCTCCATTGAGAGTTTATGTTGAAGAGTTGAAACCAACTCCT
GAAGGAGATTTGGAAATTTTGTTGCAAAAGTGGGAAAACGGAGAGTGTGCTCAAAAGAAAAT
TATCGCTGAAAAGACTAAGATCCCTGCTGTTTTTAAAATCGATGCTTTGAACGAGAATAAGGT
TTTGGTTTTGGATACTGATTACAAGAAATATTTGTTGTTCTGTATGGAAAACTCTGCTGAACC
AGAGCAATCTTTGGCTTGTCAATGTTTGGTTAGAACTCCTGAAGTTGATGATGAAGCTTTGG
AGAAGTTTGATAAAGCTTTGAAGGCTTTGCCAATGCATATCAGATTGTCTTTCAATCCTACTC
AATTGGAAGAGCAATGTCACATTGATGACGATGACAAGAAAAACCAAGATAAGACTGAAATC
CCAACTATTAATACTATTGCTTCTGGAGAGCCAACTTCTACTCCTACTATTGAAGCTGTTGAG
TCTACTGTTGCTACTTTGGAAGCTTCTCCTGAAGTTATTGAGTCTCCACCTGAGATTAATACT
GTTCAAGTTACTTCTACTGCTGTTGGTTCTGGATCCCATCACCATCACCATCACTAA (SEQ ID
NO. 19).

In some embodiments, the sequence encoding the fusion protein is operably linked to a promoter. The skilled person will be aware of suitable promoters for expression of recombinant proteins in a given host. Preferred promoters may include those which are strong constitutive promoters in the presence of a particular carbon source (e.g. glucose, glycerol) or those which are strong regulated promoters (e.g. induced or derepressed under specific conditions such as presence or absence of a particular compound).

In some embodiments, the promoter is the GAP promoter (GAPp). The GAP promoter is the promoter from the *Pichia pastoris* TDH3 gene (Glyceraldehyde-3-phosphate dehydrogenase, GAPDH). In some embodiments the promoter is that of the *Pichia pastoris* GCW14 gene (GCW14p). In some embodiments the promoter is that of the *Pichia pastoris* AOX1 gene (AOX1p) or that of the *Pichia pastoris* HSP12 gene (HSP12p, also known a DHp).

In some embodiments, the sequence further comprises a terminator. Preferred terminators may include those which are associated with high levels of expression. Suitable terminators include those of the AOX1 and TDH3 genes of *Pichia pastoris,* and the terminators of the PGK1, CYC1, RPL3, and BNA4 genes of *Saccharomyces cerevisiae.* In some embodiments, the terminator is that of the *Pichia pastoris* AOX1 gene.

The promoter, the sequence which encodes the recombinant fusion protein and the terminator (if present), which are comprised within the nucleic acid molecule, are together referred to as an "expression cassette". The invention thus also provides an expression cassette.

In some embodiments, the nucleic acid molecule further comprises one or more homology regions (e.g. two homology regions). These can be used to direct integration of the sequence encoding the recombinant fusion protein, or the expression cassette, into the genome of a host cell. The skilled person will be capable of designing suitable homology regions using their common general knowledge. For example, the homology regions may be designed based on integration loci that have been successfully used previously, loci that are known to result in higher expression, and/or loci where integration does not disturb endogenous expression.

In some embodiments, the nucleic acid molecule, or the sequence encoding the recombinant fusion protein, is codon-optimised for expression in a desired host cell, such as a bacterial cell or a eukaryotic cell (e.g. a yeast or a plant cell). In some embodiments the nucleic acid molecule, or the sequence encoding the recombinant fusion protein, is codon-optimised for expression in *Pichia pastoris.*

The invention further provides a vector comprising the nucleic acid molecule described herein.

The vector may be a plasmid, a cosmid, or a viral vector.

In some embodiments, the vector is an expression vector for expression of the recombinant fusion protein in a host cell. The expression vector may be a plasmid or a cosmid. It will be appreciated that an expression vector is a vector (e.g. a DNA vector) which can be introduced into a host cell and which will remain autonomous (i.e. it will replicate within the host cell and remain as an individual entity rather than be integrated in the genome) and from which proteins will be expressed.

In some embodiments, the vector is a cloning vector, such as a plasmid.

Also provided is a host cell comprising a nucleic acid molecule (e.g. an expression cassette) or a vector (e.g. an expression vector or a cloning vector) as described herein.

In some embodiments, the host cell comprises an expression cassette which is integrated into the genome of the host cell, wherein the expression cassette comprises a promoter, a sequence encoding the recombinant nucleic acid molecule operably linked to the promoter and, optionally, a terminator.

Integration of the expression cassette into the host cell genome can be carried out using standard techniques known to those skilled in the art, and as described herein. For example, the host cell may be co-transformed with: (i) a donor DNA molecule comprising the expression cassette located between two homology regions; and (ii) a plasmid comprising an RNA-guided endonuclease (CRISPR) system. The plasmid may further comprise an antibiotic resistance gene, enabling the selection of host cells which have been successfully transformed with the plasmid DNA. The proportion of plasmid and donor DNA in the transformation mix may be controlled so as to achieve a high probability that any host cell transformed with the plasmid is also transformed with the donor DNA molecule. The RNA-guided endonuclease (CRISPR) system ensures that the host cell genome has a double-strand cut at a target location, thereby promoting homology-driven recombination of the donor DNA molecule such that the expression cassette is integrated into the host cell genome.

In some embodiments, the host cell comprises multiple expression cassettes which are integrated into the host cell genome. In some embodiments, the host cell genome comprises two, three, four or more expression cassettes. All of the expression cassettes may be the same. For example, each expression cassette may comprise a sequence encoding the same recombinant fusion protein. Insertion of multiple copies of the same gene (i.e. the sequence encoding the recombinant fusion protein) can be useful to increase protein yield. In some embodiments, each expression cassette is different. For example, each expression cassette may comprise a sequence encoding a different recombinant fusion protein.

In embodiments wherein the host cell comprises multiple expression cassettes, each expression cassette may comprise a different promoter (and, optionally a different terminator). This may be advantageous to reduce the risk of unwanted recombinations. The relative abundance of each recombinant protein expressed may also be controlled through the use of different promoters.

Such host cells may be prepared by transforming the host cell with: (i) a first donor DNA molecule comprising a first expression cassette located between a first pair of homology regions; and (ii) a first plasmid. Once integration of the first expression cassette into the host cell genome is complete, the first plasmid can be lost by culturing the host cells without selective pressure (i.e. in the absence of the antibiotic which the first plasmid confers resistance to). The host cell can then be transformed with: (i) a second donor DNA molecule comprising a second expression cassette located between a second pair of homology regions; and (ii) a second plasmid. The first and second expression cassettes may be the same (e.g. for integration of a second copy of DNA encoding the same recombinant fusion protein) or they may be different (e.g. for integration of DNA encoding a different recombinant fusion protein). The first and second pairs of homology regions can be different, so that the first and second expression cassettes are integrated into different regions of the host cell genome. The first and second plasmids can encode different guide RNAs.

In some embodiments, the host cell comprises an expression vector. The expression vector may comprise an expression cassette comprising a promoter, a sequence encoding the recombinant nucleic acid molecule operably linked to the promoter and, optionally, a terminator. The expression vector may be a plasmid. In some embodiments, the expression vector comprises multiple expression cassette, e.g. two, three, four or more expression cassettes. The expression cassettes may all be the same, or they may be different. For example, each expression cassette may comprise a sequence encoding a different recombinant fusion protein.

The host cell may be a bacterial cell or a eukaryotic cell. A eukaryotic host can be a plant cell, an animal cell or a fungal cell. A fungal cell can be a yeast cell.

In some embodiments, the host cell is a eukaryotic cell selected from *Saccharomyces spp., Kluyveromyces spp., Pichia spp., Aspergillus spp., Tetrahymena spp., Yarrowla spp., Hansenula spp., Blastobotrys spp., Candida spp., Zygosaccharomyces spp., Debaryomyces spp., Fusarium spp., and Trichoderma spp.*

In some embodiments, the host cell is *Pichia spp,* such as *Pichia pastoris.*

In some embodiments, the host cell is a plant cell, such as soy.

The invention also provides the use of the recombinant fusion protein described herein in the production of a recombinant protein.

A method of producing a recombinant protein comprises culturing a host cell as described herein under conditions suitable for expression of a recombinant fusion protein as described herein.

Suitable conditions for culturing and expressing recombinant proteins in a given host cell will be known to those skilled in the art. For example, the expression of the recombinant fusion protein in a eukaryotic host cell, such as *Pichia pastoris,* may be carried out under the conditions described herein.

In some embodiments, a method of culturing the host cell (e.g. a yeast such as *Pichia pastoris*) comprises:
a) Culturing the host cell at a first temperature;
b) Diluting the culture; and
c) Culturing the diluted culture at a second temperature.

It will be appreciated that the first temperature will be selected according to the type of host cell. In some embodiments, the first temperature is from 25 °C to 40 °C, from 28 °C to 37 °C or from 30 °C to 35 °C. In some embodiments, the first temperature is from 28 °C to 30 °C. In certain embodiments, the first temperature is 28 °C.

The host cell may be cultured at the first temperature until mid-exponential phase. In some embodiments, the host cell is cultured at the first temperature for 6-12 hours (e.g. about 8 hours), or is cultured for a longer period (e.g. 16 hours), diluted (e.g. 1:10) and further cultured at the first temperature until mid-exponential phase (e.g. 5 hours).

In some embodiments, the culture is diluted to an OD₆₀₀ of from 0.01 to 0.3, from 0.05 to 0.25, or from 0.1 to 0.2.

In some embodiments, the second temperature is from 20 to 40 °C, from 21 to 37 °C, from 22 to 35 °C, from 23 to 30 °C or from 24 to 28 °C. In some embodiments the second temperature is 25 °C. In some embodiments the second temperature is 37 °C. In some embodiments the second temperature is 30 °C

In some embodiments, the diluted culture (or a portion thereof) is cultured for 12-30 hours, or from 16-24 hours. In some embodiments, the diluted culture (or a portion thereof) is cultured for 22 hours.

In some embodiments, the method comprises:
a) Culturing the host cell at 28 °C, optionally until the culture reaches mid-exponential phase;
b) Diluting the culture to an OD₆₀₀ of 0.2; and
c) Culturing the diluted culture at 25 °C, optionally for 16-24 hours.

In some embodiments, the host cell expresses a recombinant fusion protein which comprises a cleavage site between the first protein (e.g. BLG) and the CMP or fragment thereof. In such embodiments, the method may further comprise treating the recombinant fusion protein with an enzyme or a chemical which is capable of cleaving the recombinant fusion protein at the cleavage site, so as to separate the first protein from the CMP or fragment thereof.

In some embodiments, the method further comprises separating the recombinant fusion protein from the host cell culture, prior to treating the recombinant fusion protein with the enzyme or chemical.

Alternatively, the method may comprise treating the recombinant fusion protein with the enzyme or chemical in the host cell culture (i.e. by adding the enzyme or chemical to the culture), and then separating the cleaved first protein from the culture.

The enzyme may be any suitable enzyme, such as those described herein. In some embodiments, the enzyme is chymosin.

In some embodiments, the method may further comprise inactivating the cleaving enzyme. This may be achieved by thermally treating the enzyme e.g. by heating. For example, the method may comprise heating a suspension comprising the recombinant fusion proteins. The suspension may be the host cell culture, or it may comprise the recombinant fusion proteins after separating the proteins from the cell culture containing the enzyme. Inactivating the cleaving enzyme may help to prevent or reduce degradation of the separated first protein. In some embodiments in which the separated first protein is isolated (e.g. from the cell culture) rapidly after cleavage is performed, thermal inactivation of the cleaving enzyme may not be required.

The method may further comprise isolating and/or purifying the separated first protein (e.g. BLG).

Isolating and/or purifying the separated first protein from the host cell culture may comprise one or more of the following techniques: centrifugation; precipitation (e.g. by adjusting the pH and/or salt composition of the culture medium); ultrafiltration; size exclusion chromatography; affinity chromatography; ion exchange chromatography; reverse phase chromatography; and immunoprecipitation.

The invention thus provides a recombinant first protein obtained by the method described hereinabove. The recombinant first protein may be recombinant BLG.

It will be appreciated that the recombinant first protein may comprise, in addition to the amino acid sequence of the first protein itself, one or more additional features that were present in the recombinant fusion protein, such as one or more tags, a signal sequence fragment, and/or a linker or portion thereof.

In some embodiments, the recombinant first protein comprises at least a portion of a linker. The linker, or portion thereof, may be located at the C-terminus or the N-terminus of the recombinant first protein. In some embodiments the linker, or portion thereof, is located at the C-terminus of the recombinant first protein. In some embodiments, the recombinant first protein comprises at least a portion of a cleavage site, such as an enzyme cleavage site (e.g. a chymosin cleavage site).

In some embodiments, the recombinant first protein comprises a fragment of a signal sequence. Such a fragment may be generated by cleavage of the signal sequence during or immediately after translocation of the recombinant fusion protein into the host cell's secretory pathway. The signal sequence fragment may be located at the N-terminus of the recombinant first protein.

Thus, in some embodiments the recombinant first protein may comprise the structure:
Signal sequence fragment - first protein - linker (or portion thereof).

In some embodiments, the recombinant protein is BLG. The recombinant BLG may comprises a sequence according to SEQ ID NO: 20 or SEQ ID NO:21:

EAEALIVTQTMKGLDIQKVAGTWYSLAMAASDISLLDAQSAPLRVYVEELKPTPEGDLEILLQKW
ENGECAQKKIIAEKTKIPAVFKIDALNENKVLVLDTDYKKYLLFCMENSAEPEQSLACQCLVRTPE
VDDEALEKFDKALKALPMHIRLSFNPTQLEEQCHIHPHPHLSF (SEQ ID NO. 20).

EAEALIVTQTMKGLDIQKVAGTWYSLAMAASDISLLDAQSAPLRVYVEELKPTPEGDLEILLQKW
ENGECAQKKIIAEKTKIPAVFKIDALNENKVLVLDTDYKKYLLFCMENSAEPEQSLACQCLVRTPE
VDDEALEKFDKALKALPMHIRLSFNPTQLEEQCHIDDDDK (SEQ ID NO.21).

The invention further provides the use of the recombinant fusion protein described herein in the production of a food composition.

The invention further provides a food composition comprising a recombinant fusion protein or a recombinant first protein (e.g. recombinant BLG) as described herein.

A method of making a food composition comprises:
(i) providing a recombinant fusion protein as described herein;
(ii) separating the first protein from the CMP or fragment thereof; and
(iii) using the separated first protein to make a food composition.

In some embodiments, the recombinant fusion protein comprises a cleavage site between the first protein and the CMP or fragment thereof. In such embodiments, the step of separating the first protein from the CMP or fragment thereof comprises treating the recombinant fusion protein with an enzyme or a chemical capable of cleaving the recombinant fusion protein so as to separate the first protein from the CMP or fragment thereof.

In some embodiments, the food composition is an alternative food composition, such as an alternative dairy food composition.

As used herein, the term "alternative" refers to a composition that comprises at least one isolated, recombinant first protein, e.g. BLG. The recombinant first protein may be present in the composition instead of, or in addition to, its equivalent animal-derived protein. For example, an alternative dairy composition may comprise a recombinant BLG according to the invention instead of an animal-derived BLG. The "animal derived" will be understood as referring to a natural first protein that comes from an animal, e.g. in animal milk.

In some embodiments, the alternative food composition does not comprise any animal-derived proteins.

In some embodiments, the alternative food composition comprises one, two or three recombinant proteins in accordance with the invention

The alternative food composition may comprise one or more additional ingredients selected from: casein, α-lactalbumin, serum albumin, lactoferrin, immunoglobulin, vegetal protein, salt (e.g. sodium chloride or potassium chloride), starch, sugar (e.g. lactose, sucrose, glucose, fructose and/or galactose), water, flavouring agent, preservative, emulsifier (e.g. lecithin), fats.

In some embodiments, the alternative dairy composition is a milk composition, a butter composition, a yoghurt composition, a sour cream composition, a crème fraiche composition, a baby formula composition, a cream composition, an ice-cream composition, a custard composition, or a cheese composition.

The invention thus provides a food composition obtained by the method described herein above.

It will be appreciated that any aspect or embodiment described herein may be combined with any other aspect or embodiment as appropriate, unless otherwise stated.

### Examples

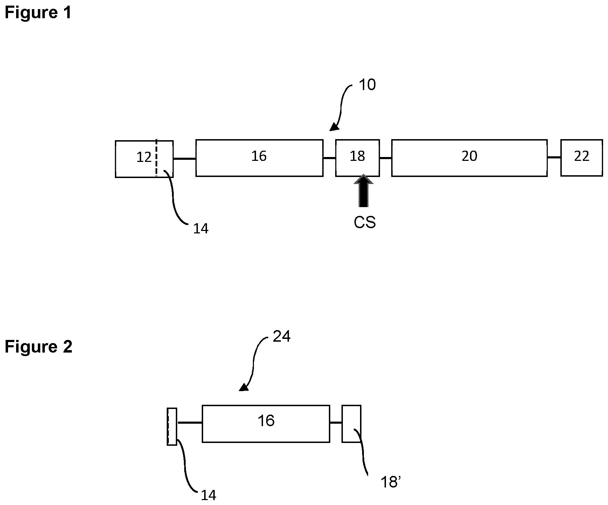

Constructs were prepared for expression of recombinant fusion proteins. With reference to Figure 1, constructs were designed to include a DNA sequence encoding a recombinant fusion protein 10 comprising, from the C-terminus to the N-terminus: a MF-a Δ57-70 signal sequence from *S*. *cerevisiae* 12; a first protein 16; a linker 18 comprising a cleavage site (CS); a caseinomacropeptide 20; and a HIS tag 22. The DNA sequence was operably linked to promoter and terminator sequences. Homology regions to direct chromosomal integration are also fused to these DNA molecules. The resulting DNA constructs were separately transformed into *Pichia pastoris* host cells, which were cultured.

It will be appreciated that in a recombinant fusion protein 24 secreted by the host cell, the signal sequence will have be cleaved (indicated by the dotted line), potentially leaving behind a short fragment of the signal sequence 14 in the expressed protein. While the fusion protein shown in the embodiment of Figure comprises a HIS tag, it will be appreciated that in other embodiments the recombinant fusion protein may contain a different tag, multiple tags or no tags.

### Comparative Example 1: Expression of recombinant casein as a CMP fusion protein

### Preparation constructs for expression of recombinant fusion proteins

1. Protein and DNA sequences of bovine caseins were obtained from databases. The codon usage of protein coding DNA was optimized for expression in *Pichia pastoris.*
2. DNA sequences coding for a promoter (GAPp: promoter of the *Pichia pastoris* TDH3 gene), *S. cerevisiae* α-mating factor (MF-a) signal sequence (SS), caseins (αS1,αS2, β, K), tags and terminator (AOX1t: terminator of the Pichia pastoris AOX1 gene) were produced by *in vitro* DNA synthesis and onboarded in a cloning vector (a derivative of pUC19 which retains the AmpR-Linker-Ori sequence, and in which the LacZ and multiple cloning site (MCS) region is replaced by a MCS partly derived from pUCmu (Staal et al. 2019 Biotechniques 66(6); 254-259) modified to include at its centre Aarl and Bsgl sites repeated and separated by a Sall site.).
3. A ca. 1000 bp region upstream of the genomic loci targeted for insertion was amplified from genomic DNA by PCR using a standard PCR protocol, thereby creating a first homology region (HRL).
4. A ca. 1000 bp region downstream of the genomic loci targeted for insertion was amplified from genomic DNA by PCR using a standard PCR protocol, thereby creating a second homology region (HRR).
   The primers used in steps 3 and 4 to create the homology regions were designed with overhangs to allow assembly of the resulting fragments and a linearized cloning vector through a standard Gibson Assembly protocol (i.e. the reverse primer for HRL has an overhang that overlaps the forward primer for HRR, the forward primer for HRL has an overhang that overlaps the reverse primer from step 5 and the reverse primer for HRR has an overhang that overlaps the forward primer from step 5). Furthermore the forward primer for HRL and the reverse primer for HRR were designed to introduce type IIS restriction sites (e.g. Aarl, Bsgl) such that recognition occurs outside the homology region but cleavage occurs within.
5. A cloning vector (pUC19) was linearized by PCR with primers that amplify the entire vector except the MCS.
6. The DNA fragments resulting from steps 3, 4 and 5 where assembled to form a circular vector pUC19-HRL-HRR by a standard Gibson Assembly protocol.
7. A DNA fragment encoding the expression cassette Promoter-SS-Casein-Tag-Terminator was amplified by PCR for each of casein αS1, αS2, β and K, from the vectors of step 2, wherein "SS" refers to a signal sequence.
8. The vector of step 6 was linearized by PCR with primers containing overhangs designed to overlap the first bases of the promoter and last bases of the terminator from step 7 (i.e. a reverse primer binding the 3' end of HRL with an overhang complementary to the 5' end of the promoter and a forward primer binding the 5' end of HRR with an overhang complementary to the 3' end of the terminator).
9. The linearized vector from step 8 was assembled into a circular vector with each of the DNA fragments from step 7 by a standard Gibson Assembly protocol, resulting in the circular vectors:
   a) pUC19-HRL-Promoter-SS-CSN1S1-Tag-Terminator-HRR
   b) pUC19-HRL-Promoter-SS-CSN1S2-Tag-Terminator-HRR
   c) pUC19-HRL-Promoter-SS-CSN2-Tag-Terminator-HRR
   d) pUC19-HRL-Promoter-SS-CSN3-Tag-Terminator-HRR
   where CSN1S1 is the gene encoding αS1 casein, CSN1S2 is the gene encoding αS2 casein, CSN2 is the gene encoding β casein, CSN3 is the gene encoding K casein and where K casein comprises the regions: para-casein K - chymosin recognition sequence (CRS) - caseinomacropeptide (CMP).
10. Vector (d) from step 9 was linearized by PCR with a reverse primer binding on the 3' end of the SS and containing an overhang coding for a tag and a forward primer binding on the 5' end of the CRS resulting in a linear DNA molecule that excludes the para Kappa Casein fragment: CRS-CM P- Tag- Terminator-H RR-pUC 19-HRL-Promoter-SS- Tag
11. DNA encoding caseins αS1, αS2, and β (without SS or Tag) was amplified by PCR from the plasmids of step 9. The primers used were designed to introduce an N-terminal tag and contain overhangs to allow Gibson Assembly with the linear DNA molecule of step 10 (i.e. a forward primer binding on the 5' end of each casein with an overhang complementary to the tag introduced by the reverse primer of step 10, and a reverse primer binding the 3' end of each casein with an overhang complementary to the 5' end of the CRS).
12. The linearized vector from step 10 was assembled into a circular vector with each of the DNA molecules from step 11 by a standard Gibson Assembly protocol resulting in the circular vectors:
   e) pUC19-HRL-Promoter-SS-Tag-CSN1S1-linker-CMP-Tag-Terminator-HRR
   f) pUC19-HRL-Promoter-SS-Tag-CSN1S2-linker-CMP-Tag-Terminator-HRR
   g) pUC19-HRL-Promoter-SS-Tag-CSN2-linker-CMP-Tag-Terminator-HRR where the linker comprises the chymosin recognition sequence (CRS).
13. Donor DNA was separated from backbone DNA (pUC19) by restriction of the vectors of steps 9 and 12 on the Aarl or Bsgl restriction sites introduced in steps 3 and 4 (on the 5' end of HRL and the 3' end of HRR) followed by agarose gel electrophoresis and extraction of the pertinent DNA band from the gel.

At all pertinent steps, correct amplification and assembly of DNA was verified by restriction analysis and/or sequencing.

Note that Gibson Assembly of only two fragments (backbone plus a single insert) can be inefficient compared to assembly of three or more fragments. To counter this, in steps 8 and 10 the backbone can be linearized into two partially overlapping fragments rather than a single full-length fragment.

### Transformation of host cells

14. Electro-competent *Pichia pastoris* cells (strain NRRL Y-11430 also known as CBS7435 or ATCC 76273) were prepared from exponentially growing cells by treatment with lithium acetate and sorbitol as described by Wu and Letchworth 2004 BioTechniques 36:152.

15. The electro-competent cells of step 14 were mixed with the donor DNA molecules of step 13 (individually) and with an episomal vector expressing an RNA guided endonuclease, guide RNA specific for the genomic loci targeted for insertion and an antibiotic resistance gene.

16. The mix of step 15 was transferred to an electroporation cuvette, subjected to a pulse (2 kV, 25 µF, 200 ohm), diluted with YPD growth media (Yeast extract 1%, Peptone 2%, Dextrose 2%), transferred to a fresh vial, allowed to recover for 2 hours at 30°C and spread on a YPD-Agar (2%) plate with an antibiotic for selection according to the resistance provided by the vector of step 15.

17. After 2-3 days of growth at 30°C single transformed colonies were picked from the selective YPD-Agar plate of step 16 and analysed by a standard colony PCR protocol with primers specific for the targeted genomic locus and the donor DNA.

18. Clones identified as positive (i.e. with a correctly integrated expression cassette) in step 17 were spread on non-selective YPD-Agar plates.

19. After 2 days at 30°C single colonies were picked from the plates of step 18 and spread on selective and non-selective plates. This step was repeated, passing cells from the non-selective plates onto selective and non-selective plates, until there was no growth on selective plates (indicating loss of the episomal vector).

### Host cell culture

20. Cells from step 19 were inoculated in 3 ml YPD and grown at 30 °C for ca. 8 h until mid-exponential phase.

21. The pre-cultures of step 20 were diluted to OD600 0.014 in 30 ml of BMDY media (2% peptone, 1% yeast extract, 100 mM potassium phosphate pH 6, 13.4 g/L YNB with ammonium sulphate, 2 % glucose 0.4 mg/L biotin) and grown for 25 h at 30 °C.

22. Samples from the cultures of step 21 where collected at various time points. Results are reported for samples at 23 hours of growth. For sampling, 4 ml was removed from the culture.

### Protein recovery

23. Secreted proteins in the samples of step 22 were separated from the cells by centrifugation, 5 min 1500 rcf.

### Determination of expression levels.

24. Recombinant casein or casein - CMP hybrids in a fraction of the supernatants from step 23 (e.g. 200 µl) were diluted as required (e.g. 500x) in PBS and quantified by a standard ELISA protocol using an HRP conjugated antibody specific against the tag (HIS), an HRP substrate (ABTS) and a calibration curve with a tagged protein of known concentration (HIS tagged FKBP12). To calculate protein concentration from detected absorbance, the differences in molecular weight of the standard and the various recombinant proteins as well as the number of tags on a single recombinant protein were taken into account.

### Results

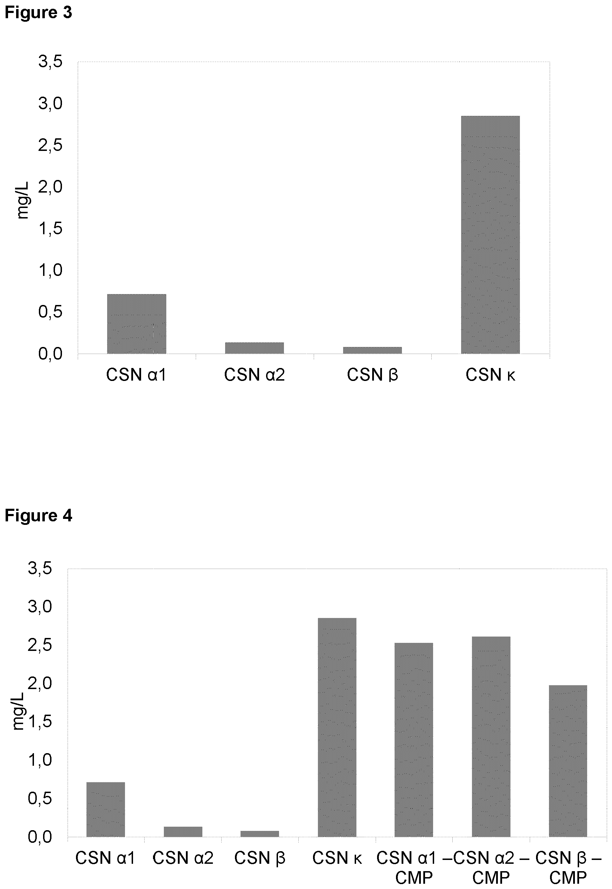

Caseins (CSN) α-S1, α-S2, β and k were expressed, under the same conditions, from a strong promoter. As shown in Figure 3, the titre of casein kappa after 23 hours of culture was found to be four times that of casein α-S1. The titres of caseins α-S2 and β were so low that they were almost at the detection limit (21 and 37 times less than that of casein kappa, respectively).

Constructs were prepared for expression of recombinant fusion proteins. Constructs were designed to include a DNA sequence encoding a recombinant fusion protein comprising, from the C-terminus to the N-terminus: a MF-a signal sequence from *S*. *cerevisiae*; a first HIS tag; a casein protein; a linker comprising a cleavage site; a caseinomacropeptide; and a second HIS tag. The DNA sequence was operably linked to promoter and terminator sequences. Homology regions to direct chromosomal integration are also fused to these DNA molecules.

Separate constructs were prepared for the expression of each of α-S1 casein, α-S2 casein and β-casein as fusion proteins.

It will be appreciated that upon secretion of the fusion proteins from the host cell the signal sequence will be cleaved, leaving behind a short fragment of the signal sequence 12' in the expressed protein. While the fusion proteins described in this example comprised HIS tags, it will be appreciated that in other embodiments the recombinant fusion protein may contain different tags or no tags.

The resulting DNA constructs were separately transformed into *Pichia pastoris* host cells, which were cultured. This resulted in the host cells expressing the recombinant fusion proteins α-S1 casein-CMP, α-S2 casein-CMP or β-casein-CMP under the same conditions and from the same promoter as the (non-fusion) recombinant caseins from Figure 3. After 23 hours the titre of casein kappa was only 1.1 times that of α-S1 casein-CMP or α-S2 casein-CMP, and 1.4 times that of β-casein-CMP (Figure 4).

### Example 1: Expression of recombinant β-lactoglobulin as a CMP fusion protein

The titre of secreted recombinant bovine BLG (Uniprot acession number P02754) and secreted recombinant BLG-CMP fusion are compared. Both proteins are expressed with a C-terminal HIS tag linked by a GSGS (SEQ ID NO: 23) linker and expressed from the same promoter and loci in cells (*Pichia pastoris*) grown under the same conditions. In this example BLG and the CMP from bovine kappa-casein (Uniprot accession number P02668) are connected by two different linkers: First, the sequence recognized and cleaved by bovine chymosin (the enzyme that cleaves the CMP in kappa-casein, Uniprot accession number P00794, EC:3.4.23.4). Second, the sequence recognized and cleaved by enteropeptidase (e.g. bovine enteropeptidase, Uniprot accession number P98072, EC:3.4.21.9). In both cases a substantial increase of titre is observed.

### Preparation of constructs

1. Protein and DNA sequences of bovine β-Lactoglobulin and kappa casein were obtained from databases. The codon usage of protein coding DNA was optimized for expression in *Pichia pastoris.*
2. DNA sequences coding for a promoter (GCW14p: promoter of the *Pichia pastoris GCW14* gene), a signal sequence (SS - *S*. *cerevisiae* α-mating factor Δ57-70 - MF-α57-70), either BLG or kappa casein (CSN3), a tag [GSGS-6xHIS (SEQ ID NO: 24)] and terminator (AOX1t: terminator of the *Pichia pastoris* AOX1 gene) were produced by *in vitro* DNA synthesis and onboarded in a cloning vector (pUC19), resulting in the circular vectors
   a) pUC19-Promoter-SS-BLG-Tag-Terminator
   b) pUC19-Promoter-SS-CSN3-Tag-Terminator
3. A ca. 1000 bp region upstream of the genomic loci targeted for insertion was amplified from genomic DNA by PCR using a standard PCR protocol, thereby creating a first homology region (HRL).
4. A ca. 1000 bp region downstream of the genomic loci targeted for insertion was amplified from genomic DNA by PCR using a standard PCR protocol, thereby creating a second homology region (HRR).
   The primers used in steps 3 and 4 to create the homology regions were designed with overhangs to allow assembly of the resulting fragments and a linearized cloning vector through a standard Gibson Assembly protocol (i.e. the reverse primer for HRL has an overhang that overlaps the forward primer for HRR, the forward primer for HRL has an overhang that overlaps the reverse primer from step 5 and the reverse primer for HRR has an overhang that overlaps the forward primer from step 5).
5. A cloning vector (pUC19) was linearized by PCR with primers that amplify the entire vector except the MCS.
6. The DNA fragments resulting from steps 3, 4 and 5 where assembled to form a circular vector by a standard Gibson Assembly protocol
   c) pUC19-HRL-HRR
7. A DNA fragment encoding the expression cassette Promoter-SS-BLG-Tag-Terminator was amplified by PCR from the vector (a) of step 2.
8. The vector (c) of step 6 was linearized by PCR with primers containing overhangs designed to overlap the first bases of the promoter and last bases of the terminator from step 7 (i.e. a reverse primer binding the 3' end of HRL with an overhang complementary to the 5' end of the promoter and a forward primer binding the 5' end of HRR with an overhang complementary to the 3' end of the terminator).
9. The linearized vector from step 8 was assembled into a circular vector with the DNA fragment from step 7 by a standard Gibson Assembly protocol, resulting in the circular vector:
   d) pUC19-HRL-Promoter-SS-BLG-Tag-Terminator-HRR
10. A DNA fragment encoding the 5' portion of the expression cassette Promoter-SS-BLG (i.e. excluding the tag and terminator) was amplified by PCR from the vector (a) of step 2.
11. A DNA fragment encoding the 3' portion of the expression cassette linker-CMP-tag-terminator (i.e. excluding promoter-SS-para-K-casein) was amplified by PCR from vector (b) of step 2. Where K-casein is para-K-casein-linker-CMP and the linker is the recognition and cleavage sequence of chymosin. The forward primer contained an overhang complementary to the 3' end of BLG.
12. The DNA fragments from steps 8, 10 and 11 where assembled into a circular vector by a standard Gibson Assembly protocol resulting in the circular vector
   e) pUC19-HRL-Promoter-SS-BLG-linker-CMP-Tag-Terminator-HRR where the linker is the recognition sequence and cleavage site of bovine chymosin.
13. A DNA fragment encoding the 3' portion of the expression cassette CMP-tag-terminator (i.e. excluding promoter-SS-para-K-casein-linker) was amplified by PCR from vector (b) of step 2. The forward primer anneals after the chymosin recognition and cleavage site, thereby excluding it. The forward primer contained an overhang corresponding to the recognition and cleavage site of enteropeptidase.
14. A DNA fragment encoding the 5' portion of the expression cassette Promoter-SS-BLG (i.e. excluding the tag and terminator) was amplified by PCR from the vector (a) of step 2. The reverse primer contained an overhang complementary to the forward primer from step 13.
15. The DNA fragments from steps 8, 13 and 14 where assembled into a circular vector by a standard Gibson Assembly protocol
   f) pUC19-HRL-Promoter-SS-BLG-linker-CMP-Tag-Terminator-HRR where the linker is the recognition and cleavage sequence of enteropeptidase.
16. Donor DNA was prepared from the vectors (d), (e) and (f) of steps 9, 12 and 15 by a standard PCR reaction with a forward primer annealing at the 5' end of HRL and a reverse primer annealing at the 3' end of HRR followed by agarose gel electrophoresis and extraction of the pertinent DNA band from the gel.

At all pertinent steps, correct amplification and assembly of DNA was verified by restriction analysis and/or sequencing.

### Transformation of host cells

17. Electro-competent *Pichia pastoris* cells (strain NRRL Y-11430 also known as CBS7435 or ATCC 76273) were prepared from exponentially growing cells by treatment with lithium acetate and sorbitol as described by (Wu, S. and Letchworth G.J. High efficiency transformation by electroporation of Pichia pastoris pretreated with lithium acetate and dithiothreitol 2004 BioTechniques 36:152).

18. The electro-competent cells of step 17 were mixed with the donor DNA molecules of step 16 (individually) and with an episomal vector expressing an RNA guided endonuclease, guide RNA specific for the genomic loci targeted for insertion and an antibiotic resistance gene.

19. The mix of step 18 was transferred to an electroporation cuvette, subjected to a pulse (2 kV, 25 µF, 200 ohm), diluted with YPD growth media (Yeast extract 1%, Peptone 2%, Dextrose 2%), transferred to a fresh vial, allowed to recover for 2 hours at 30°C and spread on a YPD-Agar (2%) plate with an antibiotic for selection according to the resistance provided by the vector of step 18.

20. After 2-3 days of growth at 30°C single transformed colonies were picked from the selective YPD-Agar plate of step 19 and analysed by a standard colony PCR protocol with primers specific for the targeted genomic locus and the donor DNA.

21. Clones identified as positive (i.e. with a correctly integrated expression cassette) in step 20 were spread on non-selective YPD-Agar plates.

22. After 2 days at 30°C single colonies were picked from the plates of step 21 and spread on selective and non-selective plates. This step was repeated, passing cells from the non-selective plates onto selective and non-selective plates, until there was no growth on selective plates (indicating loss of the episomal vector).

### Host cell culture

23. Cells from step 22 were inoculated in 3 ml YPD and grown at 28 °C for ca. 16 h, cultures were then diluted 1:10 in YPD and grown until mid-exponential phase (ca. 5 h) at 28 °C.
- 2 independent clones transformed with donor DNA from vector (d), i.e. expressing BLG-Tag.
- 2 independent clones transformed with donor DNA from vector (e), i.e. expressing BLG-linker(chym)-CMP-Tag where linker(chym) is the recognition and cleavage sequence of bovine chymosin.
- 1 clone transformed with donor DNA from vector (f), i.e. expressing BLG-linker(entp)-CMP-Tag where linker(entp) is the recognition and cleavage sequence of enteropeptidase.

24. The pre-cultures of step 23 were diluted to OD600 0.2 in 30 ml of BMDY media (2% peptone, 1% yeast extract, 100 mM potassium phosphate pH 6, 13.4 g/L YNB with ammonium sulphate, 2 % glucose 0.4 mg/L biotin) and grown for 22 h at 25 °C (ca. OD600 20).

25. Samples from the cultures of step 24 where collected by removing 4 ml from the culture.

### Protein recovery

26. Secreted proteins in the samples of step 25 were separated from the cells by centrifugation, 5 min 1500 rcf.

### Protein Visualisation and Determination of expression levels.

### SDS-PAGE - WB

27. An aliquot (e.g. 2.5 ul) of the supernatant from step 26 was mixed with (e.g. 0.5 ul) 6X Laemmli sample buffer with 0.6 M dithiothreitol (DTT) and incubated at 65°C for 10 minutes.

28. The samples from step 27 were separated through a standard SDS-PAGE electrophoresis protocol and analysed through a standard western blot protocol with an antibody specific for the tags (HIS)

### ELISA

29. Recombinant BLG or BLG-CMP fusion in a fraction of the supernatants from step 26 (e.g. 200 µl) were diluted as required (e.g. 500x) in PBS and quantified by a standard ELISA protocol using an HRP conjugated antibody specific against the tag (HIS), an HRP substrate (ABTS) and a calibration curve with a tagged protein of known concentration (HIS tagged FKBP12). To calculate protein concentration from detected absorbance, the differences in molecular weight of the standard and the different recombinant proteins were taken into account.

### Results

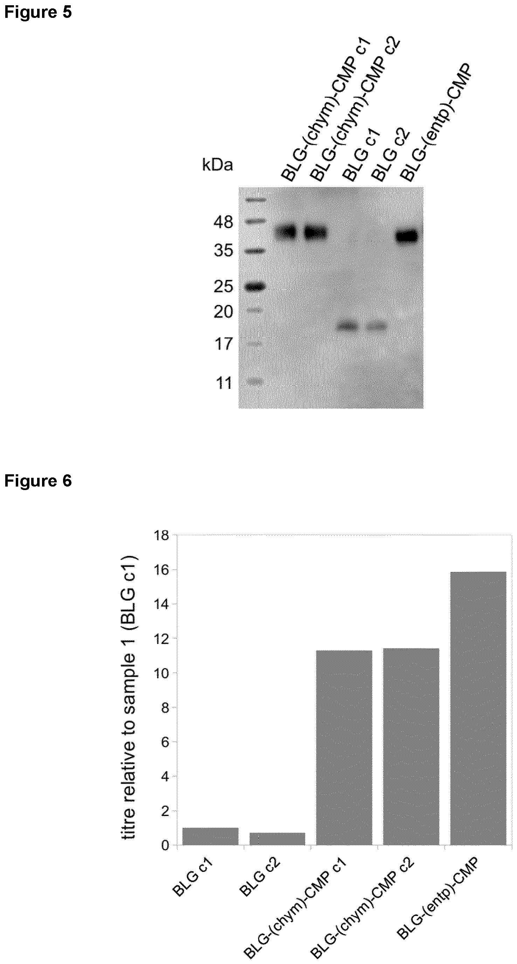

With reference to Figure 5, recombinant BLG is seen on the western blot as a single sharp band at the expected size (under 20 kDa). The fusion BLG-CMP is also seen as a single sharp band. Due to glycosylation of the CMP the fusion protein appears at a higher molecular weight (above 35 kDa) than the 26 kDa predicted by the amino acid sequence alone.

Bands of BLG-CMP are much more intense than bands of BLG, indicating that the fusion protein is produced with higher titre.

The results of the ELISA (Figure 6) corroborate this. The BLG-CMP fusion proteins are produced with a titre between 11 and 16 fold that of the non-fusion BLG.

### Abbreviations

- ABTS: 2,2'-azino-bis(3-ethylbenzothiazoline-6-sulfonic acid)
- BLG: β-Lactoglobulin
- CMP: Caseinomacropeptide
- DTT: Dithiothreitol
- ELISA: Enzyme-Linked Immunosorbent Assay
- HRL: Homology Region Left
- HRR: Homology Region Right
- HRP: Horseradish Peroxidase
- PBS: Phosphate Buffer Saline
- PCR: Polymerase Chain Reaction
- rcf: Relative Centrifugal Force
- SDS-PAGE: Sodium Dodecyl Sulfate - Polyacrylamide Gel Electrophoresis
- SS: Signal Sequence
- WB: Western Blot
- YPD: Yeast Extract, Peptone, Dextrose

The invention may be described by any of the following clauses:
1. A recombinant fusion protein comprising:
   (a) a first protein; and
   (b) a caseinomacropeptide (CMP), or a fragment thereof,
   wherein the first protein is not a casein.
2. The recombinant fusion protein of clause 1, wherein the recombinant fusion protein comprises a sequence of no more than 150, no more than 120, no more than 100, no more than 90, no more than 80, no more than 75, no more than 70, no more than 65 or no more than 60 consecutive amino acids derived from the C-terminus of κ-casein.
3. The recombinant fusion protein of any preceding clause, wherein the CMP or fragment thereof is capable of decreasing the hydrophobicity of the recombinant fusion protein relative to the hydrophobicity of the first protein alone.
4. The recombinant fusion protein of any preceding clause, wherein the solubility of the recombinant fusion protein in a medium is greater than the solubility of the first protein in the medium.
5. The recombinant fusion protein of any preceding clause, wherein the CMP or fragment thereof comprises an amino acid sequence of at least 20 residues.
6. The recombinant fusion protein of any preceding clause, wherein the CMP comprises an N-terminal and/or a C-terminal truncation.
7. The recombinant fusion protein of any preceding clause, wherein the CMP or fragment thereof comprises:
   (i) the amino acid sequence:
      X₃SGX₂PX₁ X₁X₁PTX₃X₂X₃X₃X₂S X₁X₃X₃X₁X₃X₂X₃X₁PX₂ X₃ (SEQ ID NO:3)
      wherein
      each X₁ is independently selected from S and T,
      each X₂ is independently selected from D and E, and
      each X₃ is independently selected from A, I, L and V, or
   (ii) an amino acid sequence which is at least 80% identical to SEQ ID NO: 3, optionally wherein at least 3 of the X₁ residues are retained.
8. The recombinant fusion protein of any preceding clause, wherein the CMP or fragment thereof comprises
   (i) an amino acid sequence selected from:
      X₃SGX₂PX₁ X₁X₁PTX₃X₂X₃X₃X₂S X₁X₃X₃X₁X₃X₂X₃X₁PX₂ X₃X₃X₂SPPX₂X₃NT X₃QX₃X₁STX₃X₃ (SEQ ID NO:4); and
      KNQX₂KTX₂X₃PX₁ X₃NTX₃X₃SGX₂PX₁ X₁X₁PTX₃X₂X₃X₃X₂S X₁X₃X₃X₁X₃X₂X₃X₁PX₂ X₃ (SEQ ID NO:5), or
   (ii) an amino acid sequence which is at least 80% identical to SEQ ID NO:4, optionally wherein at least 3 of the X₁ residues are retained, or
   (iii) an amino acid sequence which is at least 80% identical to SEQ ID NO:5, optionally wherein at least 3 of the X₁ residues are retained.
9. The recombinant fusion protein of any preceding clause, wherein the CMP or fragment thereof comprises
   (i) the amino acid sequence 
KNQX2KTX2X3PX1 X3NTX3X3SGX2PX1 X1X1PTX3X2X3X3X2S X1X3X3X1X3X2X3X1PX2
X3X3X2SPPX2X3NT X3QX3X1STX3X3 (SEQ ID NO:6);
 or
   (ii) an amino acid sequence which is at least 80% identical to SEQ ID NO:6, optionally wherein at least 3 of the X₁ residues are retained.
10. The recombinant fusion protein of part (ii) or (iii) of any one of clauses 7 to 9, wherein all of the X₁ residues are retained.
11. The recombinant fusion protein of any preceding clause, wherein the CMP or fragment thereof comprises
   (i) the amino acid sequence ASGEPT STPTIEAVES TVATLEASPE V (SEQ ID NO:7), or
   (ii) an amino acid sequence which is at least 80% identical to SEQ ID NO: 7.
12. The recombinant fusion protein of any preceding clause, wherein the CMP or fragment thereof comprises:
   (i) an amino acid sequence selected from:
      ASGEPT STPTIEAVES TVATLEASPE VIESPPEINT VQVTSTAV (SEQ ID NO:8);
      KNQDKTEIPT INTIASGEPT STPTIEAVES TVATLEASPE V (SEQ ID NO:9); and
      KNQDKTEIPT INTIASGEPT STPTIEAVES TVATLEASPE VIESPPEINT VQVTSTAV (SEQ ID NO:10), or
   (ii) an amino acid sequence which is at least 80% identical to SEQ ID NO:8, SEQ ID NO:9 or SEQ ID NO:10.
13. The recombinant fusion protein of any preceding clause wherein the CMP or fragment thereof comprises at least 3 glycosylation sites.
14. The recombinant fusion protein of any preceding clause wherein the CMP or fragment thereof is capable of increasing the yield of the recombinant fusion protein relative to the yield of the equivalent first protein alone when expressed under identical conditions.
15. The recombinant fusion protein of any preceding clause wherein the first protein is a contractile, structural, storage or transport protein, optionally wherein the first protein is a storage protein.
16. The recombinant fusion protein of clause 15, wherein the storage protein is a milk protein, an egg protein or a plant storage protein.
17. The recombinant fusion protein of clause 16, wherein the storage protein is selected from the proteins listed in Table 1.
18. The recombinant fusion protein of clause 17, wherein the storage protein is β-lactoglobulin (BLG).
19. The recombinant fusion protein of any preceding clause, which further comprises a linker between the first protein and the CMP or a fragment thereof.
20. The recombinant fusion protein of clause 19, wherein the linker comprises a cleavage site, optionally an enzyme cleavage site.
21. The recombinant fusion protein of clause 20, wherein the enzyme cleavage site is a protease cleavage site, optionally a chymosin cleavage site.
22. A nucleic acid molecule comprising a sequence encoding the recombinant fusion protein of any one of claims 1 to 21.
23. A vector comprising the nucleic acid molecule of clause 22.
24. A host cell comprising the nucleic acid of clause 22 or the vector of clause 23.
25. The host cell of clause 24, wherein the host cell is a plant cell, a bacterial cell, a fungal cell, a yeast cell or a mammalian cell, optionally wherein the yeast cell is *Pichia pastoris.*
26. The use of a recombinant fusion protein according to any one of clauses 1 to 21 in the production of a recombinant protein.
27. The use of a recombinant fusion protein according to any one of clauses 1 to 21 in the production of a food composition.
28. A method of producing a recombinant protein, the method comprising culturing the host cell of claim 24 under conditions suitable for expression of the recombinant fusion protein of any one of claims 1 to 21.
29. The method of clause 28, wherein the recombinant fusion protein comprises a linker comprising a cleavage site, and wherein the method further comprises treating the recombinant fusion protein with an enzyme or a chemical which is capable of cleaving the recombinant fusion protein at the cleavage site, so as to separate the first protein from the CMP or fragment thereof.
30. A recombinant protein obtained by the method of clause 29, optionally wherein the recombinant protein comprises at least a portion of a cleavage site.
31. A method of making a food composition, the method comprising:
   (i) providing a recombinant fusion protein according to any one of clauses 1 to 21;
   (ii) separating the first protein from the CMP or fragment thereof; and
   (iii) using the separated first protein to make a food composition.
32. A food composition obtained by the method of any one of clauses 28 to 31.
33. A food composition comprising a recombinant fusion protein according to any one of clauses 1 to 21, or a recombinant protein according to clause 30.

## Claims

1. A recombinant fusion protein comprising:
(a) a first protein; and
(b) a caseinomacropeptide (CMP), or a fragment thereof,
wherein the first protein is not a casein.

2. The recombinant fusion protein of claim 1, wherein the recombinant fusion protein comprises a sequence of no more than 150, no more than 120, no more than 100, no more than 90, no more than 80, no more than 75, no more than 70, no more than 65 or no more than 60 consecutive amino acids derived from the C-terminus of κ-casein, optionally wherein the CMP or fragment thereof comprises an amino acid sequence of at least 20 residues.

3. The recombinant fusion protein of claim 1 or claim 2, wherein the CMP or fragment thereof comprises an amino acid sequence selected from Group A, Group B and Group C:
**Group A**
(i) the amino acid sequence:
X₃SGX₂PX₁ X₁X₁PTX₃X₂X₃X₃X₂S X₁X₃X₃X₁X₃X₂X₃X₁PX₂ X₃ (SEQ ID NO:3) or
(ii) an amino acid sequence which is at least 80% identical to SEQ ID NO: 3, optionally wherein at least 3 of the X₁ residues are retained;
or
**Group B**
(i) an amino acid sequence selected from:
X₃SGX₂PX₁ X₁X₁PTX₃X₂X₃X₃X₂S X₁X₃X₃X₁X₃X₂X₃X₁PX₂ X₃X₃X₂SPPX₂X₃NT X₃QX₃X₁STX₃X₃ (SEQ ID NO:4); and
KNQX₂KTX₂X₃PX₁ X₃NTX₃X₃SGX₂PX₁ X₁X₁PTX₃X₂X₃X₃X₂S X₁X₃X₃X₁X₃X₂X₃X₁PX₂ X₃ (SEQ ID NO:5), or
(ii) an amino acid sequence which is at least 80% identical to SEQ ID NO:4, optionally wherein at least 3 of the X₁ residues are retained, or
(iii) an amino acid sequence which is at least 80% identical to SEQ ID NO:5, optionally wherein at least 3 of the X₁ residues are retained;
or
**Group C**
(i) the amino acid sequence 
KNQX2KTX2X3PX1 X3NTX3X3SGX2PX1 X1X1PTX3X2X3X3X2S X1X3X3X1X3X2X3X1PX2
X3X3X2SPPX2X3NT X3QX3X1STX3X3 (SEQ ID NO:6);
 or
(ii) an amino acid sequence which is at least 80% identical to SEQ ID NO:6, optionally wherein at least 3 of the X₁ residues are retained;
wherein
each X₁ is independently selected from S and T,
each X₂ is independently selected from D and E, and
each X₃ is independently selected from A, I, L and V.

4. The recombinant fusion protein of any preceding claim, wherein the CMP or fragment thereof comprises an amino acid sequence selected from Group E or Group F:
**Group E**
(i) the amino acid sequence
ASGEPT STPTIEAVES TVATLEASPE V (SEQ ID NO:7), or
(ii) an amino acid sequence which is at least 80% identical to SEQ ID NO: 7;
or
**Group F**
(i) an amino acid sequence selected from:
ASGEPT STPTIEAVES TVATLEASPE VIESPPEINT VQVTSTAV (SEQ ID NO:8);
KNQDKTEIPT INTIASGEPT STPTIEAVES TVATLEASPE V (SEQ ID NO:9); and
KNQDKTEIPT INTIASGEPT STPTIEAVES TVATLEASPE VIESPPEINT VQVTSTAV (SEQ ID NO:10), or
(ii) an amino acid sequence which is at least 80% identical to SEQ ID NO:8, SEQ ID NO:9 or SEQ ID NO:10.

5. The recombinant fusion protein of any preceding claim, which further comprises a linker between the first protein and the CMP or fragment thereof, wherein the linker comprises a cleavage site, optionally an enzyme cleavage site.

6. The recombinant fusion protein of any preceding claim, wherein the first protein is a contractile, structural, storage or transport protein, optionally wherein the first protein is a storage protein

7. The recombinant fusion protein of claim 6, wherein the storage protein is a milk protein, an egg protein or a plant storage protein, optionally wherein the storage protein is selected from the proteins listed in Table 1.

8. The recombinant fusion protein of claim 7, wherein the storage protein is β-lactoglobulin (BLG).

9. A nucleic acid molecule comprising a sequence encoding the recombinant fusion protein of any one of claims 1 to 7.

10. A vector comprising the nucleic acid molecule of claim 9.

11. A host cell comprising the nucleic acid of claim 9 or the vector of claim 10, wherein the host cell is a plant cell, a bacterial cell, a fungal cell, a yeast cell or a mammalian cell, optionally wherein the host cell is *Pichia pastoris.*

12. A method of producing a recombinant protein, the method comprising culturing the host cell of claim 11 under conditions suitable for expression of the recombinant fusion protein of any one of claims 1 to 8.

13. A recombinant protein obtained by the method of claim 12, optionally wherein the recombinant protein comprises at least a portion of a cleavage site, further optionally wherein the recombinant protein is β-lactoglobulin (BLG).

14. A method of making a food composition, the method comprising:
(i) providing a recombinant fusion protein according to any one of claims 1 to 8;
(ii) separating the first protein from the CMP or fragment thereof; and
(iii) using the separated first protein to make a food composition.

15. A food composition obtained by the method of claim 14.
